# EUROPEAN PATENT SPECIFICATION

(11) **EP 0 465 979 B1**
(45) Date of publication and mention of the grant of the patent: **05.11.1997**
(21) Application number: 91110884.3
(22) Date of filing: 01.07.1991
(51) Int. Cl.: C12P 21/08, C12N 5/20, A61K 39/395, G01N 33/569, G01N 33/577

(54) **Anti HTLV-III (strain MN) monoclonal antibody**
Monoklonaler Antikörper gegen HTLV-III (Stamm MN)
Anticorps monoclonal contre l'HTLV-III (souche MN)

(30) Priority: 02.07.1990 JP 175075/90; 16.07.1990 JP 188300/90
(43) Date of publication of application: 15.01.1992
(73) Proprietor: Juridical Foundation The Chemo-Sero-Therapeutic Research Institute, Kumamoto-shi Kumamoto-ken (JP)
(72) Inventor: Eda, Yasuyuki, Kikuchi-gun, Kumamoto-ken (JP); Osatomi, Kiyoshi, Kumamoto-shi, Kumamoto-ken (JP); Shiosaki, Kouichi, Kikuchi-gun, Kumamoto-ken (JP); Tokiyoshi, Sachio, Kumamoto-shi, Kumamoto-ken (JP); Matsushita, Shuzo, Kumamoto-shi, Kumamoto-ken (JP); Hattori, Toshio, Kumamoto-shi, Kumamoto-ken (JP); Takatsuki, Kiyoshi, Kumamoto-shi, Kumamoto-ken (JP)
(74) Representative: VOSSIUS & PARTNER

(56) References cited:
- EP-A- 339 504
- WO-A-88/09181
- WO-A-90/03984
- WO-A-90/15078
- JOURNAL OF IMMUNOLOGY, vol. 144, no. 4, 15 February 1990, Baltimore, MD (US); Y.-W. KIM et al., pp. 1257-1262
- JOURNAL OF IMMUNOLOGY, vol. 144, no. 12, 15 June 1990, Baltimore, MD (US); A. PROFY et al., pp. 4641-4647
- JOURNAL OF IMMUNOLOGY, vol. 142, no. 10, 15 May 1989, Baltimore, MD (US); T. PALKER et al., pp. 3612-3619
- AIDS RESEARCH & HUMAN RETROVIRUSES, vol. 6, no. 3, March 1990, New York, NY (US); Y. DEVASH et al., pp. 307-316

## Description

This invention relates to an immunological technology providing a novel substance for the prophylaxis, treatment and diagnosis of virus infectious diseases. More particularly, it relates to a monoclonal antibody being capable of substantially neutralizing human immunodeficiency virus (abbreviated as HIV) which is the etiologic agent of the acquired immunodeficiency syndrome (abbreviated as AIDS), and to a hybridoma being capable of secreting the monoclonal antibody.

HIV is a retrovirus which is known to be the cause of diseases such as AIDS and AIDS-related complex (abbreviated as ARC). It is well known that proto-type HIV includes human T-lymphotropic virus type III (abbreviated as HTLV-III) and lymphadenopathy associated virus (abbreviated as LAV). The above diseases are one of the recent most serious problems in the world, and it has been desired to develop a vaccine or a therapeutic method for the treatment thereof, but there has never been found any effective means. The most characteristic hematological anomaly in AIDS is functional and quantitative loss of helper/inducer T lymphocyte having CD4 antigen on the surface thereof. The immunodeficiency caused by HIV induces various disorders in the bio-phylactic mechanism in the infected host (human), and then highly frequently induces opportunistic infections such as Pneumocystis carinii pneumonia and unusual malignant tumors such as Kaposi's sarcoma. The immunodeficiency caused by HIV is a progressive and irreversible disease with high death rate, and it is considered that the death rate of the disease will reach 100 % within several years.

In case of infection of HIV to T cell with virus particles, the virus particles will first bind to the receptor CD4 antigen. The infection of HIV also spreads via cell-to-cell infection. That is, infected cells are cell-fused with non-infected cells, and particularly in organs such as brain, lymphonodes, etc., syncytium (macropolycyte) is formed. The syncytium formation is also observed in experiment in vitro. It is usually considered that the T cells infected with HIV are easily suffered from cytopathic effect of HIV and this will cause the loss of CD4-positive cell.

It is also known that HIV infects not only the helper/inducer T lymphocytes but also monocyte/macrophages and further that most monocyte/macrophages and a part of the T lymphocytes have resistance to the cytopathic effect of HIV and hence these cells retain the virus for a long period of time and continuously produce the virus.

Moreover, it is known that human blood serum infected by HIV contains an antibody to HIV, but the antibody has merely low neutralizing activity (cf. Weiss et al., Nature, 316, p.69-72, 1985).

It is well known that a core antigen (gag) and an envelope antigen are present as the structural protein antigen of HIV. The HIV viral envelope protein is expressed as a precursor glycoprotein having a molecular weight of 160 kilodaltons (gp160) that is proteolytically cleaved to generate an external envelope glycoproteins having a molecular weight of 120 kilodaltons (gp120) and a transmembrane envelope glycoprotein having a molecular weight of 41 kilodaltons (gp41). Among these, gp120 is the most important by the following reasons.
(1) When a test animal is immunized with the gp120 or with a certain fragment derived from the gp120, a polyclonal neutralizing antibody is produced. This means that the gp120 is at least one of the target molecules of an antibody capable of neutralizing the virus (cf. Lasky et al., Science, 233, p.209-212, 1986).
(2) At the first step of infection of HIV, the gp120 binds to CD4 molecule of virus receptor. This means that the gpl20 is the most important molecule as to the HIV infection (McDougal et al., Science 231, p.382-385, 1986).
(3) The syncytium formation by HIV, that is cell-to-cell infection of HIV, is induced by the direct interaction between the gp120 and the CD4 molecule of non-infected, cells (cf. Lifson et al., Nature, 323, p.725-728, 1985).

Various monoclonal antibodies to constructive proteins of HTLV-IIIB or LAV have been known, for example, antibody against p24 which is one of core antigens present within virus (Veronese, F.D., Proc. Natl. Acad. Sci., U.S.A., 82, p.5199-5202, 1985); antibody to pol gene product encoding a reverse transcriptase of virus (Veronese, F.D., Science, 231, p.1289-1291, 1986); and antibody to gp41 which is another constructive protein in the envelope (Veronese, F.D., Science, 229, p.1402-1405, 1985). However, none of these known monoclonal antibodies does react with the gp120 antigen which is an important factor for the prophylaxis and treatment of AIDS. It is rather reported that any monoclonal antibody being capable of effectively neutralizing HIV could not be obtained even by immunizing animals with a purified LAV (Chassange, J. et al., J. Immunol., 136, p.1442-1445, 1985).

There have hitherto been studied various methods for obtaining monoclonal antibody being capable of effectively neutralizing AIDS virus and hence being useful for the prophylaxis, treatment and diagnosis of AIDS.

It is reported that a monoclonal antibody to the gp120 antigen has been obtained by using a synthetic peptide as an immunogen and that an epitope recognized by the antibody is within the region of the amino acid sequence 503-532 of the HIV envelope (Chanh, T.C. et al., Eur. J. Immunol., 16, p.1455-1468, 1986). However, the antibody had very weak binding activity as indicated both in Western blotting method and in immunofluorescent method. In this report, no evidence is shown of the presence of the neutralizing activity of said monoclonal antibody.

The present inventors have also obtained a monoclonal antibody (0.5β) being capable of effectively neutralizing the virus by binding to the gp120 of HTLV-III_{B} strain (Matsushita et al., J. Virology, 62, p.2107-2114, 1988). However, said 0.5β antibody can neutralize HTLV-III_{B} strain, but not HTLV-III_{MN} strain which is more popular in immunological field.

WO 90/03984 relates to the identification of a portion of the HIV-1 (HTLV-III) envelope protein gp120 recognized as the principal neutralizing domain. The precise location of said domain on the gpl20 of 16 various HIV-1 strains including the MN strain is shown and polypeptides comprising this domain and having the capability of raising, and/or binding with, neutralizing monoclonal and polyclonal antibodies are disclosed. Four peptides comprising segments of the principal neutralizing domain from HIV-1MN are specifically disclosed. Peptide 142 has the amino acid sequence Tyr Asn Lys Arg Lys Arg Ile His Ile Gly Pro Gly Arg Ala Phe Tyr Thr Thr Lys Asn Ile Ile Gly.

The present inventors have found a monoclonal antibody which can bind to the envelope antigen of HTLV-III_{MN}: gp120 and can substantially neutralize the virus.

An object of the invention is to provide a monoclonal antibody being capable of substanially neutralizing HIV. Another object of the invention is to provide a hybridoma being capable of producing said monoclonal antibody. These and other objects and advantages of the invention will be apparent to those skilled in the art from the following description.

The figures show:

Fig. 1 shows a reactivity of the monoclonal antibodies of the invention (µ39.1 and µ5.5) to the synthetic peptides of gp120 (amino acid sequence 303-325 or 308-329) derived from the various HIV mutants. An initial concentration of each antibody is 500 µg/ml.

Fig. 2 shows a reactivity of the monoclonal antibodies of the invention (µ39.1 and µ5.5) to the external envelope glycoprotein gp120 derived from the HTLV-III_{MN}-infected cells.

The term "neutralization" in this disclosure means inhibition of cell free infection of HIV and also cell-to-cell infection such as syncytium formation which occurs between HIV-infected cells and non-infected cells by interaction between the gp120 and the CD4.

This invention provides a monoclonal antibody which is produced by the hybridoma FERM BP-3402 or a fragment thereof, said monoclonal antibody being capable of binding to a glycoprotein antigen having a molecular weight of about 12 x 10⁴ daltons (gp120) present in the envelope of human T-lymphotropic virus III_{MN} (HTLV-III_{MN}), being capable of substantially neutralizing HTLV-III_{MN}, and has the following characteristics:
(a) immunoglobulin class: IgG, k
(b) being capable of binding to glycoprotein antigen having a molecular weight of 12 x 10⁴ daltons (gp120) of HTLV-III_{MN},
(c) being capable of recognizing at least one epitope which is present in the range of the amino acid sequence 303 to 325 (Tyr Asn Lys Arg Lys Arg Ile His Ile Gly Pro Gly Arg Ala Phe Tyr Thr Thr Lys Asn Ile Ile Gly) of gp120 of HTLV-III_{MN},
(d) being capable of binding to the surface of HTLV-III_{MN} viral particles and thereby inhibiting the infection of the virus particles to CD4-positive cells, and
(e) being capable of binding to the surface of cells infected with HTLV-III_{MN} and thereby inhibiting the syncytium formation induced by interaction between the infected cells and uninfected cells.

Thus, the monoclonal antibody of this invention can clearly inhibit the cell-to-cell infection such as syncytium formation and/or cell-free virus infection such as infection with HTLV-III_{MN}. Accordingly, the monoclonal antibody can be used for the prophylaxis and treatment of AIDS. Moreover, the monoclonal antibody of this invention is also useful for the inhibition of growth of AIDS virus in human host. Besides, since the monoclonal antibody of this invention has a strong neutralizing activity against HTLV-III_{MN}, it is also effective for the prevention of infection of the virus to uninfected T cells.

The hybridoma being capable of producing the monoclonal antibody of this invention has been deposited to Fermentation Research Institute, Agency of Industrial Science and Technology, Tsukuba, Japan under Budapest Treaty in accession No. FERM BP-3402 on July 10, 1990. The said hybridoma is also referred to hereafter under the designation "µ5.5".

This invention is illustrated by the following Examples but should not be construed to be limited thereto.

### Example 1

Preparation of monoclonal antibody:

### Preparation of antigen

### (1) A synthetic peptide:

A synthetic peptide corresponding to the amino acid sequence 303 to 325 of the envelope glycoprotein gp120 of HTLV-III_{MN} (Tyr Asn Lys Arg Lys Arg Ile His Ile Gly Pro Gly Arg Ala Phe Tyr Thr Thr Lys Asn Ile Ile Gly) is used as an immunogen and an antigen for assay.

The above peptide is prepared with ABI430A Peptide Synthesizer (Applied Biosystem). The crude peptide thus prepared is removed from the substrate resin by TFMSA method (Yanaihara, C., Experimental Medicine, 6, No. 10, p.141-148, 1988) and purifed by reverse phase high performance liquid chromatography (HPLC). The purification by reverse phase HPLC is repeated three times and the fractions containing the product are collected, and the product is subjected to amino acid analysis, by which it is confirmed that the amino acid sequence of the product corresponds well to that of HTLV-III_{MN} strain, and thereby it is concluded that the product is a synthetic peptide of gp120 of HTLV-III_{MN} strain.

The thus-obtained synthetic peptide (designated "SP-1") is lyophilized, and then is bound to an immunization carrier, KLH (Keyhole Limpet Hemocyanin) to give a peptide-KLH conjugate in the following manner.

That is, the above peptide SP-1 (10 mg) is dissolved in 10 mM phosphate buffered saline (PBS, pH 7.0, 2 ml), and thereto is added a solution of MBS crosslinking agent in dimethylformamide (40 mg/100 µl), and the mixture is stirred at room temperature for 30 minutes. The reaction mixture is washed with dichloromethane (2 ml) three times, and the aqueous layer (designated "Solution A") is separated.

Separately, KLH (20 mg) is dissolved in 0.2 M Tris-HCl buffer (pH 8.6, 8 M urea, 5 ml) and thereto is added dithiothreitol (DTT), and the mixture is stirred at room temperature for one hour. To the reaction mixture is added 10 % trichloroacetic acid (3 ml), and the resulting precipitate is separated by filtration with suction, washed with distilled water (2 ml) and then dissolved in 20 mM sodium phosphate buffer (NaPB, pH 7.0, 0.6 M urea, 5 ml) to give a solution (Solution B).

The above Solution A and Solution B are mixed and stirred at room temperature for 3 hours, and the reaction product is dialyzed and lyophilized.

The synthetic peptide of gp120 of HTLV-III_{MN} strain and peptide-KLH conjugate prepared above are used as an immunogen and antigen for assay.

### (2) Cultivation of HTLV-III_{MN}-producing cells and preparation of HTLV-III_{MN} particles:

H9/HTLV-III_{MN} strain is used as HTLV-III_{MN}-producing cells. A culture medium is RPMI 1640 supplemented with 20 % FCS and 2 mM L-glutamine to be used in a 50 L scale. The H9/HTLV-III_{MN} strain is cultivated in said culture medium in a 36 liter Spinner flask with a cultivation controller (manufactured by Wakenyaku Kogyo K.K.) and the resulting cells-floating mixture is centrifuged at 3,000 r.p.m. for 5 minutes to separate the culture supernatant. The culture supernatant is subjected to sucrose density-gradient centrifugation (25 %, 50 %, discontinuing, 89,000 x g, 20 h.) with a continuous rotater (RPC35T, manufactured by Hitachi Ltd.) at a rate of 2 liter/h. to separate viral particles, wherein the viral particles are collected in 30 - 45 % sucrose layer. The viral particles thus obtained are used as an immunogen and an antigen for assay.

Purified gp120 is prepared by collecting the cells from the above H9/HTLV-III_{MN} culture broth by centrifugation, lysing the cells with 1 % Triton® X-100, centrifuging the mixture and then purifying the supernatant by affinity chromatography with ConA - Sepharose® 4B column. The eluted solution is further purified by affinity chromatography with HIV antibody (IgG) - Sepharose® 4B column. The purified gp120 thus obtained is used as an immunogen and an antigen for assay.

### (3) Preparation of recombinant expression peptide of HTLV-III_{MN} gp120 V3 domain:

H9/HTLV-III_{MN} cells (10⁶ - 10⁷ cells) are floated in 1 x RSB buffer and thereto are added sodium dodecylsulfate (SDS, at final concentration of 1 %) and Proteinase K (at final concentration of 1 mg/ml), and the mixture is incubated at 37°C for 2 hours. The resulting mixture is repeatedly subjected to extraction with phenol and precipitation with ethanol to give a high molecular weight DNA (genomic DNA). HTLV-III_{MN} gp120 V3 domain (amino acid 247 - 370) is amplified by conventional PCR method by using a template of the above high molecular weight DNA and the following A primer and C primer: 
A primer: (5')TGTACACATGGAATTAGGCCAG(3')
C primer: (3')GAAGTCCTCCCCTGGGTCTTTA(5')
 The amplification is carried out with Taq polymelase for 30 to 35 cycles.

The amplified DNA fragment is cloned with pUC18 plasmid, and the cloned DNA fragment is inserted into pUEX2 expression vector (manufactured by Amersham, code No. RPN1515; Bressan, G. and Stanley, Y., Nucleic Acid Research, 15, p.10056, 1987). Escherichia coli is transfected with the expression vector and then subjected to heat induction at 42°C to express the peptide. The expressed HTLV-III_{MN} gp120 V3 domain (amino acid 247 - 370) is a fusion protein with β-galactosidase, which is then purified in the form of E. coli-inclusion body as follows.

After expression, E. coli is fractured with glass beads and treated with lysozyme (final concentration, 0.1 mg/ml) at 4°C. The resulting precipitate separated by centrifugation is treated with Triton® X-100 (final concentration, 0.5 %). The precipitate is solubilized with 8 M urea and is used as an immunogen and an antigen for assay.

### Immuno-sensitization of mouse

An example of immuno-sensitization of mouse with the synthetic peptides prepared hereinabove is illustrated below.

BALB/c mice (4 - 8 weeks age) are inoculated with the synthetic peptide and synthetic peptide-KLH conjugated antigen mixture (each 100 µg) three times in intraperitoneal route and one time in intravenous route, on the first day i.p. in the presence of Freund's complete adjuvant, on 14th day i.p. in the presence of Freund's incomplete adjuvant, on 28th day i.p. in the presence of Freund's incomplete adjuvant, and on 42nd day i.v. in the absence of an adjuvant.

### Cell fusion and cultivation of hybridoma

Three days after the immunization, the spleen cells are collected from the mice in a usual manner.

The spleen cells are mixed with myeloma cells p3X63Ag8-Ul in a ratio of cells of 1 : 5, and the mixture is centrifuged (1,200 r.p.m./5 minutes) to remove the supernatant. The precipitated mass of cells is well untangled and is added to a mixture (1 ml) of polyethylene glycol-4000 (2 g), minimum essential medium (MEM) (2 ml) and dimethylsulfoxide, and the mixture is incubated at 37°C for 5 minutes, and thereto is slowly added MEM so as to be total volume 50 ml. The mixture is centrifuged (900 r.p.m./5 minutes) to remove the supernatant fluid and the cells are untangled mildly. To the cells is added a normal medium (RPMI1640 medium with 10 % FCA) (100 ml), and the cells are gradually suspended therein with a measuring pipette.

The suspension is poured into each well of a 24-well culture plate (1 ml/well) and the plate is incubated in an incubator containing 5 % CO₂ at 37°C for 24 hours. Then, 1 ml/well of HAT medium [a normal medium supplemented with hypoxanthine (1 x 10⁻⁴ M), thymidine (1.5 x 10⁻³ M) and aminopterin (4 x 10⁻⁷ M)] is added and the plate is incubated for additional 24 hours. The culture is continued for 10 to 14 days in the same manner while exchanging the culture supernatant (1 ml) with the same volume of a HT medium (HAT medium depleted with aminopterin) every 24 hours for 2 days.

Each well with the fused cells (about 300 cells) growing in a colonial shape is selected. The culture supernatant (1 ml) of the selected well is exchanged with the same volume of the HT medium and then the exchange is repeated every 24 hours for 2 days.

After 3 to 4 day culture with the HT medium, a part of the culture supernatant is collected and used for selection of the desired hybridoma by screening method as described hereinbelow.

### Screening of hybridoma

The desired hybridoma is selected by a combination of enzyme immunoassay (EIA), immunofluorescence and Western blotting methods. The thus selected clone is measured for its neutralizing activity.

### (1) EIA:

To each well of a 96-well microtest plate is added 100 µl/well of the synthetic peptide antigen, purified gp120 antigen, or recombinant peptide (protein concentration: 2 µg/ml), prepared as mentioned above, and the plate is incubated at 4°C overnight for immobilization. Then, 2 % bovine serum albumin (BSA) solution (100 µl) is added to each well and the plate is incubated in the same manner for masking. To each well of the thus prepared antigen-immobilized plate are added the hybridomas obtained by the cell fusion and the culture supernatant of hybridomas after cloning and the plate is incubated at 37°C for 2 hours. The plate is washed with 0.1 % Tween® 20/PBS three times and 100 µl/well of a solution of peroxidase-labelled anti-mouse immunoglobulin (manufactured by Cappel, x 5,000 dilution). After incubation at 37°C for 1 hour, the plate is washed with 0.1% Tween® 20/PBS five times. Then, a substrate solution of 3,3',5,5'-tetramethylbenzidine (TMBZ) is added to each well for color development and an optical density is measured at 450 nm. A hybridoma clone is thus selected which strongly reacts only with the synthetic peptide derived from HTLV-III_{MN} but not with the synthetic peptide derived from HTLV-III_{B}.

### (2) Immunofluorescence:

H9/HTLV-III_{MN} cells or uninfected H9 cells (5 x 10⁵ cells) suspended in the culture supernatant to be tested (100 µl) are cultured at 4°C for 30 minutes. The cultured cells are washed twice with a PBS solution containing BSA (2 %) and azide (0.1 %) (PBS-BSA-Az). After washing, 100 µl of anti-mouse IgG labelled with fluorescein-isothiocyanate (FITC) (manufactured by Sigma, diluted to 1:40 with PBS-BSA-Az) and the mixture is reacted at 4°C for 30 minutes. The reaction mixture is washed with PBS-BSA-Az three times and then fixed with PBS containing 0.1 % paraformaldehyde.

Using a laser flow-cytometry (Spectrum III manufactured by Ortho Diagnostics), the reactivity of the antibody is measured based on the strength of fluorescence. A hybridoma showing a maximum binding ability to the surface of H9/HTLV-III_{MN} cells is selected and cloned by limiting dilution method. The hybridoma clone after cloning is also selected in the same manner.

### (3) Western blotting:

Western blotting is carried out in accordance with Towbin et al. [Proc. Natl. Acad. Sci. U.S.A., 76, p4350 (1979)].

A purified HTLV-III_{MN} virus is prepared by the method described in the literature [Science, 224, p.497 (1984)] and electrophoresed by 12 % sodium dodecyl sulfate-polyacrylamide gel electrophoresis (SDS-PAGE). The gel is then transferred to nitrocellulose membrane to transfer the virus to the membrane and the membrane is cut into strips with 0.4 to 0.5 cm width. Each strip is immersed in a hybridoma culture supernatant and incubated at room temperature overnight. After washing with PBS three times, each strip is warmed in a solution of biotin-labelled anti-mouse IgG (manufactured by TAGO) diluted to 1:750. After washing with PBS three times, each strip is immersed in a solution of horseradish peroxidase-conjugated avidin (manufactured by Sigma) diluted to 1:1000 and warmed for 1 hour. After washing with PBS three times, a coloring reagent containing 4-chloro-1-naphthol (manufactured by Bio-Rad) is used for color development. A hybridoma showing a colored band of HTLV-III_{MN} gp120 is selected and cloned. The hybridoma clone after cloning is also selected in the same manner.

### (4) Measurement of neutralizing activity:

The culture supernatant of H9/HTLV-III_{MN} is used as an original viral solution (10^{4.5} to 10⁵ TCID₅₀).

The viral solution adjusted to 10 TCID₅₀/50 µl and 50 µl of the hybridoma clone culture supernatant or purified ascites, which are diluted in series, are inoculated into each well of a 96-well flat-bottomed plate and the plate is incubated at 37°C for 1 hour. Then, MT4 cells are added to each well at 10⁴ cells/100 µl/well, said cells being floated in RPMI 1640 medium supplemented with 10 % FCS, L-glutamine (3.5 to 4.0 g/l), penicillin (50 U/ml) and streptomycin (50 µg/ml), and cultured at 37°C for 5 days.

The neutralizing activity is evaluated based on an ability of the antibody to inhibit the syncytium formation observed during infection. The neutralization titer is expressed as a minimum effective concentration of the antibody showing 100 % inhibition of syncitium formation.

The above selection procedure provides hybridomas (µ39.1 and µ5.5) capable of producing the desired monoclonal antibody.

### Preparation of monoclonal antibodies with hybridomas µ39.1 and µ5.5:

Each 5 x 10⁶ cells/animal of the hybridoma µ39.1 or µ5.5 obtained above is intraperitoneally administered to pristane-treated female BALB/c mice (8 weeks age). After 10 to 21 days, ascites cancer is induced. Ascites are taken out from the mice and centrifuged at 3,000 rpm for 5 minutes to remove solid components. Then, the antibody is purified by subjecting the supernatant to affinity chromatography using Affigel Protein A MAPS-II Kit (manufactured by Bio-Rad).

### Example 2

### Analysis of monoclonal antibodies µ39.1 and µ5.5

### (1) Reactivity to gp120 synthetic peptide derived from various HIV mutants:

Synthetic peptides of gp120 (amino acid sequence 303-325 or 308-329) derived from HTLV-III_{MN}, HTLV-III_{B}, HTLV-III_{RF}, and HIV-2 are employed. The reactivity is tested in the same manner as described in the above Screening of hybridoma, (1) EIA.

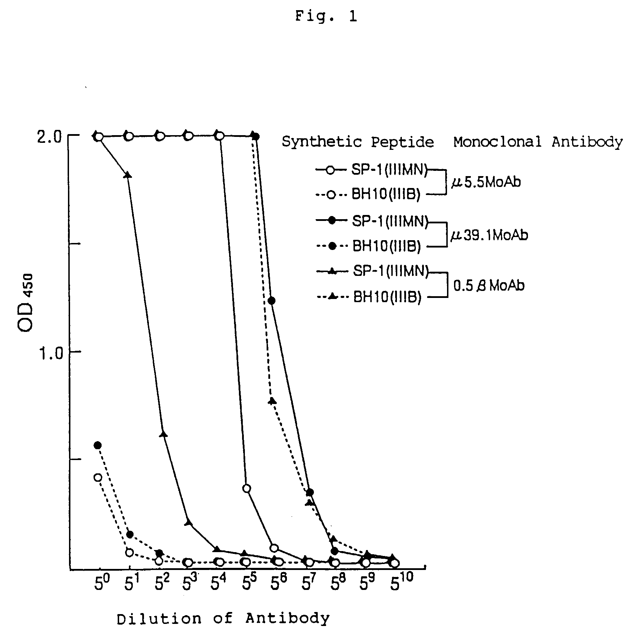

As shown in Fig. 1, it is clear that the control 0.5β antibody strongly reacts with the peptide derived from HTLV-III_{B} but not with the peptide derived from HTLV-III_{MN} at a lower concentration although it cross-reacts with the peptide derived from HTLV-III_{MN} at a higher concentration.

On the other hand, it is seen that the monoclonal antibody µ39.1 is a HTLV-III_{MN}-specific antibody which strongly reacts with the peptide derived from HTLV-III_{MN}. It is also seen that the µ39.1 monoclonal antibody reacts neither with the synthetic peptides derived from HTLV-III_{RF} nor with those from HIV-2 (data is not shown in Fig. 1).

The reactivity of the monoclonal antibody µ5.5 is completely the same as that of µ39.1, i.e. this monoclonal antibody is a HTLV-III_{MN}-specific antibody which strongly reacts only with the peptide derived from HTLV-III_{MN}.

### (2) Reactivity to gp120 derived from infected cells (Western blotting):

In order to determine the reactivity of the monoclonal antibodies µ39.1 and µ5.5 to the external envelope glycoprotein gp120 derived from infected cells, Western blotting technique is used. An antigen used is an H9/HTLV-III_{MN} cell lysate. The procedure described in the above Screening of hybridoma, (3) Western blotting is repeated.

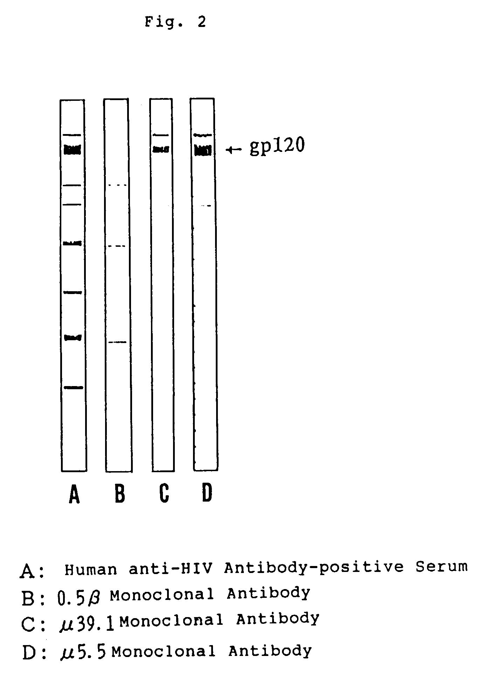

As shown in Fig. 2, strip A is a positive control in which HIV antibody positive human serum is employed, wherein a gp120 band is observed. The monoclonal antibody 0.5β does not react with gp120 derived from HTLV-III_{MN} (strip B) while the monoclonal antibodies µ39.1 and µ5.5 recognize gp120 derived from HTLV-III_{MN} (strips C and D). It is also found that the reactivity of the monoclonal antibody µ5.5 is stronger than that of the monoclonal antibody µ39.1 as shown in Fig. 2.

### (3) Neutralizing property of monoclonal anbibodies µ39.1 and µ5.5:

The neutralizing property of the monoclonal antibodies µ39.1 and µ5.5 is examined according to the procedure described in the above Screening of hybridoma, (4) measurement of neutralizing activity. The results are shown in the following Table 1.

**Table 1**

| | Inhibitory activity on cell to cell infect. by infected cells¹ | | | Virus-neutralizing activity² | | |
|---|---|---|---|---|---|---|
| MoAb | µ5.5 | µ39.1 | 0.5β | µ5.5 | µ39.1 | 0.5β |
| Virus | | | | | | |
| III_{MN} | 16 | 63 | >500 | 1 | 63 | >500 |
| III_{B}/LAV | >500 | >500 | 31 | >500 | >500 | 4 |
| III_{RF} | >500 | >500 | >500 | >500 | >500 | >500 |

| | | | | | | |
|---|---|---|---|---|---|---|
| (Note): 1. Minimum effective concentration (µg/ml) of the antibody showing 80 % inhibition of cell to cell infection by infected cells | | | | | | |
| 2. Minimum effective concentration (µg/ml) of the antibody showing 100 % inhibition of viral infection | | | | | | |

The right column in Table 1 shows a minimum effective concentration of the antibody showing 100 % inhibition of infection of each variant viral species. The control monoclonal antibody 0.5β shows a neutralizing activity specific to HTLV-III_{B}/LAV. On the other hand, the monoclonal antibody µ39.1 is a monoclonal antibody capable of specifically neutralizing HTLV-III_{MN} which inhibits the infection of HTLV-III_{MN} totally (100 %) at a concentration of 63 µg/ml but not the infection of the other HTLV strains III_{B} and III_{RF}. The monoclonal antibody µ5.5, likewise µ39.1, shows a neutralizing activity sepecfic to the strain III_{MN}. It is seen that the neutralizing activity of the monoclonal antibody µ5.5 is more than 50 times higher than that of µ39.1 and is a strong neutralizing antibody which inhibit the infection of the strain III_{MN} totally (100 %) at a concentration of 1 µg/ml.

The left column of Table 1 indicates a minimum effective concentration of the antibody showing 80 % inhibition of cell to cell infection by infected cells. The control monoclonal antibody 0.5β shows a neutralizing activity specific to III_{B}/LAV infected cells. On the other hand, the monoclonal antibody µ39.1 inhibits the cell to cell infection by III_{MN} infected cells at a concentration of 63 µg/ml but not the infection by III_{B} or III_{RF} infected cells. That is, it is found that the monoclonal antibody µ39.1 is a neutralizing antibody specific to the strain III_{MN} in the cell to cell infection by the infected cells.

The monoclonal antibody µ5.5, likewise µ39.1, also shows a neutralizing activity specific to the strain III_{MN}. It is seen that the neutralizing activity of the monoclonal antibody µ5.5 is more than about 4 times higher than that of µ39.1 and is a strong neutralizing antibody which inhibit the cell to cell infection by the infected cells at a concentration of 16 µg/ml.

## Claims

1. A monoclonal antibody produced by the hybridoma FERM BP-3402 or a fragment thereof, said monoclonal antibody being capable of binding to a glycoprotein antigen having a molecular weight of about 12 x 10⁴ daltons (gp120) present in the envelope of human T-lymphotropic virus III_{MN} (HTLV-III_{MN}), being capable of substantially neutralizing HTLV-III_{MN} and having the following characteristics:
(a) immunoglobulin class: IgG, k
(b) it is capable of recognizing at least one epitope which is present in the region of the amino acid sequence 303 to 325 (Tyr Asn Lys Arg Lys Arg Ile His Ile Gly Pro Gly Arg Ala Phe Tyr Thr Thr Lys Asn Ile Ile Gly) of gp 120 of HTLV-III_{MN};
(c) it is capable of binding to the surface of HTLV-IIIMN viral particles and thereby inhibiting the infection of the viral particles to CD4-positive cells; and
(d) it is capable of binding to the surface of cells infected with HTLV-III_{MN} and, thereby inhibiting the syncytium formation induced by interaction between the infected cells and uninfected cells.

2. A pharmaceutical composition containing the monoclonal antibody or fragment thereof according to claim 1, optionally in combination with a pharmaceutically acceptable carrier and/or diluent.

3. The pharmaceutical composition of claim 2 for the prophylaxis and/or treatment of AIDS.

4. A diagnostic composition containing the monoclonal antibody or fragment thereof according to claim 1.

5. The diagnostic composition of claim 4 for the detection of HIV or fragments thereof.

6. A test kit for the diagnosis of HIV or fragments thereof containing the monoclonal antibody or fragment thereof according to claim 1.

## Patentansprüche

1. Monoclonaler Antikörper, produziert von dem Hybridom FERM BP-3402, oder ein Fragment davon, wobei der monoclonale Antikörper fähig ist zur Bindung an ein Glycoproteinantigen mit einem Molekulargewicht von etwa 12 x 10⁴ Daltons (gp120), das auf der Hülle des menschlichen T-lymphotropen Virus III_{MN} (HTLV-III_{MN}) vorkommt, und im wesentlichen zur Neutralisation von HTLV-III_{MN} und die folgenden Eigenschaften aufweist:
(a) Immunglobulinklasse: IgG, k;
(b) er ist fähig zur Erkennung von mindestens einem Epitop, das im Bereich der Aminosäuresequenz 303 bis 325 (Tyr Asn Lys Arg Lys Arg Ile His Ile Gly Pro Gly Arg Ala Phe Tyr Thr Thr Lys Asn Ile Ile Gly) von gp 120 von HTLV-III_{MN} vorhanden ist;
(c) er ist fähig zur Bindung an die Oberfläche von HTLV-III_{MN}-Viruspartikeln und dadurch zur Hemmung einer Viruspartikelinfektion von CD4-positiven Zellen; und
(d) er ist fähig zur Bindung an die Oberfläche von mit HTLV-III_{MN} infizierten Zellen und dabei zur Hemmung der Syncytiumbildung, die durch Wechselwirkung zwischen den infizierten und den nicht-infizierten Zellen induziert wird.

2. Arzneimittel, enthaltend den monoclonalen Antikörper oder ein Fragment davon nach Anspruch 1, gegebenenfalls in Verbindung mit einem pharmazeutisch verträglichen Träger und/oder Verdünnungsmittel.

3. Arzneimittel nach Anspruch 2 zur Prophylaxe und/oder Behandlung von AIDS.

4. Diagnostische Zusammensetzung, enthaltend den monoclonalen Antikörper oder ein Fragment davon nach Anspruch 1.

5. Diagnostische Zusammensetzung nach Anspruch 4 zum Nachweis von HIV oder Fragmenten davon.

6. Testkit zur Diagnose von HIV oder Fragmenten davon, enthaltend den monoclonalen Antikörper oder ein Fragment davon nach Anspruch 1.

## Revendications

1. Anticorps monoclonal produit par l'hybridome FERM BP-3402 ou fragment de cet anticorps, ledit anticorps monoclonal étant capable de se lier à un antigène glycoprotéique ayant une masse moléculaire d'environ 12x10⁴ Da (gp120) présent dans l'enveloppe du virus HTLV-III_{MN} (human T-lymphotropic virus III_{MN}) et de neutraliser sensiblement le HTLV-III_{MN} et présentant les caractéristiques suivantes :
(a) classe d'immunoglobuline : IgG, k ;
(b) il est capable de reconnaître au moins un épitope présent dans la région de la séquence d'acides aminés 303 à 325 (Tyr Asn Lys Arg Lys Arg Ile His Ile Gly Pro Gly Arg Ala Phe Tyr Thr Thr Lys Asn Ile Ile Gly) de la gp120 de HTLV-III_{MN} ;
(c) il est capable de se lier à la surface des particules virales de HTLV-III_{MN} et d'inhiber ainsi l'infection des cellules CD4-positives par les particules virales ; et
(d) il est capable de se lier à la surface de cellules infectées par HTLV-III_{MN} et d'inhiber ainsi la formation du syncytium induite par l'interaction entre cellules infectées et cellules non infectées.

2. Composition pharmaceutique contenant l'anticorps monoclonal ou un fragment de ce dernier selon la revendication 1, facultativement en association avec un véhicule et/ou diluant pharmaceutiquement acceptables.

3. Composition pharmaceutique selon la revendication 2, destinée à la prévention et/ou au traitement du SIDA.

4. Composition diagnostique contenant l'anticorps monoclonal ou un fragment de ce demier selon la revendication 1.

5. Composition diagnostique selon la revendication 4, destinée à la détection du VIH ou ses fragments.

6. Kit de dosage destiné au diagnostic du VIH ou de ses fragments, contenant l'anticorps monoclonal ou un fragment de ce dernier selon la revendication 1.
